# EUROPEAN PATENT SPECIFICATION

(11) **EP 4 483 715 B1**
(45) Date of publication and mention of the grant of the patent: **30.09.2026**
(21) Application number: 23781345.6
(22) Date of filing: 29.03.2023
(51) Int. Cl.: A01N 63/22, A01N 37/44, A01P 3/00, C12N 1/20, C12R 1/07

(54) **COMPOSITION CONTAINING BACILLUS AMYLOLIQUEFACIENS STRAIN FOR ENHANCING PLANT DISEASE RESISTANCE AND USE THEREOF**
ZUSAMMENSETZUNG MIT BACILLUS-AMYLOLIQUEFACIENS-STAMM ZUR ERHÖHUNG DER RESISTENZ GEGEN PFLANZENKRANKHEITEN UND VERWENDUNG DAVON
COMPOSITION CONTENANT UNE SOUCHE DE BACILLUS AMYLOLIQUEFACIENS POUR AMÉLIORER LA RÉSISTANCE DES PLANTES AUX MALADIES ET SON UTILISATION

(30) Priority: 30.03.2022 KR 20220039717
(43) Date of publication of application: 01.01.2025
(73) Proprietor: CJ CheilJedang Corporation, Seoul 04560 (KR)
(72) Inventor: SONG, Gun Chul, Seoul 04560 (KR); YUN, Yeo Hong, Seoul 04560 (KR); LEE, Mi Rong, Seoul 04560 (KR)
(74) Representative: Meissner Bolte Partnerschaft mbB
(86) International application number: PCT/KR2023/004170
(87) International publication number: WO 2023/191490

(56) References cited:
- WO-A1-2016/109396
- KR-A- 20120 004 856
- KR-A- 20120 004 856
- KR-A- 20140 051 698
- KR-A- 20150 049 136
- KR-A- 20170 100 644
- KR-B1- 102 324 419
- KR-B1- 102 324 419
- US-A1- 2020 100 504

## Description

### [Technical Field]

The present disclosure relates to a composition for enhancing plant disease resistance, the composition including a *Bacillus amyloliquefaciens* microorganism which is deposited with Accession No. KCCM13138P, and/or a culture thereof; a composition for controlling a plant disease; a method of enhancing plant disease resistance using the same; a method of preventing or treating a plant disease; a method of controlling a plant disease; a *Bacillus amyloliquefaciens* microorganism which is deposited with Accession No. KCCM13138P; use of the microorganism or composition in enhancing plant disease resistance; use in controlling a plant disease; and use in preventing or treating a plant disease.

### [Background Art]

Chemical pesticides and fertilizers have greatly contributed to freeing humanity from hunger by increasing agricultural productivity. Agricultural and horticultural fungicide/insecticide including some compounds have been studied (US 10123537 B2), but the negative aspects of toxicity to human and livestock and environmental pollution have been recently receiving more social attention than the positive effects. Accordingly, there is a growing interest in eco-friendly pest control agricultural methods of growing crops by reducing the use of synthetic pesticides or without using pesticides.

Among them, biological controls using microorganisms or plant extracts for agricultural crop pest control have received attention. These biological controls may be used in place of chemical pesticides that are harmful to the environment, thereby providing consumers with safe agricultural products.

### [Disclosure]

### [Technical Problem]

The problem to be solved in the present disclosure is to provide a composition for enhancing plant disease resistance, the composition including a *Bacillus amyloliquefaciens* microorganism which is deposited with Accession No. KCCM13138P; a composition for controlling a plant disease; a method of enhancing plant disease resistance using the same; a method of preventing or treating a plant disease; a method of controlling a plant disease; a *Bacillus amyloliquefaciens* microorganism which is deposited with Accession No. KCCM13138P; use of the microorganism or composition in enhancing plant disease resistance; use in controlling a plant disease; and use in preventing or treating a plant disease.

### [Technical Solution]

An object of the present disclosure is to provide a composition for enhancing plant disease resistance and a composition for controlling a plant disease, each composition including a *Bacillus amyloliquefaciens* microorganism which is deposited with Accession No. KCCM13138P and/or a culture thereof.

Another object of the present disclosure is to provide a composition for enhancing plant disease resistance and a composition for controlling a plant disease, each composition including a *Bacillus amyloliquefaciens* microorganism which is deposited with Accession No. KCCM13138P and valine.

Still another object of the present disclosure is to provide a method of enhancing plant disease resistance using the composition for enhancing plant disease resistance.

Still another object of the present disclosure is to provide a method of preventing or treating a plant disease using the composition for enhancing plant disease resistance.

Still another object of the present disclosure is to provide a method of controlling a plant disease using the composition for controlling a plant disease.

Still another object of the present disclosure is to provide a *Bacillus amyloliquefaciens* microorganism which is deposited with Accession No. KCCM13138P.

Still another object of the present disclosure is to provide use of the *Bacillus amyloliquefaciens* microorganism which is deposited with Accession No. KCCM13138P in enhancing plant disease resistance, controlling a plant disease, and preventing or treating a plant disease.

Still another object of the present disclosure is to provide use of the composition including the *Bacillus amyloliquefaciens* microorganism which is deposited with Accession No. KCCM13138P and valine in enhancing plant disease resistance, controlling a plant disease, and preventing or treating a plant disease.

### [Advantageous Effects]

There is an effect of enhancing disease resistance of a plant body by using *Bacillus amyloliquefaciens* N351 strain which is deposited with Accession No. KCCM13138P. Further, disease resistance of the plant body may be more effectively enhanced by using a mixture of the strain and valine.

### [Description of Drawings]

FIGS. 1A and 1B show that a *Bacillus amyloliquefaciens* N351 strain has excellent disease control effects, as compared to other strains of the genus *Bacillus* and a product control, through images of leaves and results of measuring lengths of symptoms;
FIGS. 2A and 2B show the microbicidal effects (plant disease control effects) of the *Bacillus amyloliquefaciens* N351 strain against various plant pathogens, through petri-dish images of examining zone of inhibition and results of measuring the diameter of zone of inhibition;
FIG. 3 shows the biofilm formation effect of the *Bacillus amyloliquefaciens* N351 strain;
FIGS. 4A and 4B show the excellent disease control effect of the *Bacillus amyloliquefaciens* N351 strain using images of leaves through a hyperspectral camera and a visible light band camera and NDVI values;
FIGS. 5A to 5C show the excellent disease control effects of the *Bacillus amyloliquefaciens* N351 strain against soybean Phytophthora stem and root rot, cucumber bacterial leaf blight, and lettuce bacterial soft rot using disease severity, and control values;
FIGS. 6A to 6E show pot experiments for the excellent disease control effects of a *Bacillus amyloliquefaciens* N351 strain powder preparation against rice blast, tomato gray mold, wheat leaf rust, barley powdery mildew, and pepper anthracnose;
FIGS. 7A and 7B show pot experiments for the disease control effects of a mixture of the *Bacillus amyloliquefaciens* N351 strain powder preparation and valine against soybean Phytophthora stem and root rot and cucumber bacterial leaf blight;
FIG. 8 shows glass greenhouse experiments for the disease control effects of the mixture of the *Bacillus amyloliquefaciens* N351 strain powder preparation and valine;
FIGS. 9A and 9B show the induced resistance ability of the mixture of the *Bacillus amyloliquefaciens* N351 strain powder preparation and valine, through pathogen population and GUS activity; and
FIGS. 10A and 10B show the plant growth-promoting ability of the mixture of the *Bacillus amyloliquefaciens* N351 strain powder preparation and valine in soybeans and cucumbers.

### [Best Mode for Carrying Out the Invention]

Hereinafter, the present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Furthermore, the scope of the present disclosure is not limited by the specific description described below. Further, a number of papers and patent documents are referenced and cited throughout this specification.

An aspect of the present disclosure provides a composition for enhancing plant disease resistance, the composition including any one or more of a *Bacillus amyloliquefaciens* microorganism which is deposited with Accession No. KCCM13138P and a culture thereof.

In the present disclosure, the *Bacillus amyloliquefaciens* microorganism which is deposited with Accession No. KCCM13138P is named N351 CP02-1001, and may be used interchangeably with *Bacillus amyloliquefaciens* N351 microorganism, *Bacillus amyloliquefaciens* N351 strain, N351 microorganism, N351 strain, *Bacillus amyloliquefaciens* N351 CP02-1001 microorganism, *Bacillus amyloliquefaciens* N351 CP02-1001 strain, N351 CP02-1001 microorganism, and N351 CP02-1001 strain.

In one embodiment, the N351 microorganism may have the ability to enhance plant disease resistance.

In one embodiment, the N351 microorganism may have the ability to control a plant disease.

In one embodiment, the N351 microorganism may have the ability to prevent or treat a plant disease.

In one embodiment, the N351 microorganism may form a biofilm.

In one embodiment, the N351 microorganism may have the more excellent ability to enhance plant disease resistance, to control a plant disease, and/or to form a biofilm than control strains of *Bacillus amyloliquefaciens, Bacillus velezensis,* and *Bacillus siamensis* taxonomically belonging to the *Bacillus amyloliquefaciens* group; and *Bacillus subtilis,* but is not limited thereto. The control strains may be *Bacillus amyloliquefaciens* KACC10116, *Bacillus velezensis* QST713, and *Bacillus siamensis* KACC15859, and the *Bacillus subtilis* may be *Bacillus subtilis* KACC10854PB3.

In one embodiment, the N351 microorganism may be used for enhancing plant disease resistance, for controlling a plant disease, and/or for preventing or treating a plant disease in any one or more selected from the group consisting of legumes, fruit vegetables, leafy vegetables, root vegetables, and grains, but is not limited thereto.

In one embodiment, the N351 microorganism may have the ability to prevent or treat a plant disease or to enhance plant disease resistance, the plant disease caused by infection with any one or more pathogens selected from the group consisting of *Phytophthora sojae, Pseudomonas syringae pv. lachrimans, Pectobacterium carotovorum subsp. Carotovorum, Magnaporthe oryzae, Botrytis cinerea, Puccinia recondite, Blumeria graminis f. sp. hordei, Colletotrichum coccodes, Pseudomonas syringae pv. tomato, Rhizoctonia solani, Pythium ultimum, Colletotrichum truncatum, Colletotrichum acutatum, Sclerotina sclerotiorum, Phytophthora capsici, Phytophthora infestans, Fusarium oxysporum,* and *Pseudomonas syringae pv. tabaci,* but is not limited thereto.

In one embodiment, the N351 microorganism may produce various secondary metabolites, may be heat-stable, and may form endospores resistant to adverse environments, but is not limited thereto.

The culture of the present disclosure may be a medium obtained by culturing a specific microorganism in a culture medium, a culture solution, an extract, a diluent, a concentrate, a filtrate, a dry product, a lysate obtained by collecting and disrupting cells of the microorganism, a fermented product, a purified product, and/or any type of formulation which may be formed using the same, and the culture medium means that it contains a specific microorganism, and the culture filtrate means that it substantially does not contain the specific microorganism (here, it means that the specific microorganism to be separated by filtration, etc. is substantially excluded, but does not mean that the microorganism is completely excluded from the filtrate.).

In the present disclosure, the "plant disease resistance" refers to the property of preventing the invasion of pathogens, the property of preventing the development of a plant disease, and/or the property of not getting sick even when pathogens invade. The plant disease resistance may include induced resistance, but is not limited thereto. Plants have an immune system that resists various attacks by pathogens, and this immune system may exhibit disease resistance responses not only in leaves invaded but also throughout the entire plant body that has not been invaded. This immune system is called induced resistance, and use thereof may overcome the shortcomings of biological control agents and may maximize efficacy thereof.

In one embodiment, the composition for enhancing plant disease resistance of the present disclosure may induce systemic induced resistance of plants, but is not limited thereto.

In one embodiment, the composition for enhancing plant disease resistance of the present disclosure may enhance plant disease resistance, and at the same time, may not inhibit plant growth, but is not limited thereto. Specifically, as substances that induce plant resistance, pathogens as well as derivatives that induce various resistance responses are known, but some of the substances (e.g., benzo(1,2,3)-thiadiazole-7-carbothioic acid S-methyl ester, benzothiadiazole; BTH) have serious side effects such as inhibiting plant growth or significantly reducing yields. However, the composition for enhancing plant disease resistance of the present disclosure does not inhibit the plant growth, but rather may enhance the plant growth.

**In** one embodiment, the composition for enhancing plant disease resistance of the present disclosure may prevent or treat a plant disease caused by infection of a plant with a pathogen, and may induce systemic induced resistance of a plant, but is not limited thereto.

**In** the present disclosure, the "preventing" means all actions by which the infection with pathogens, or occurrence of a plant disease or development thereof is restrained or retarded.

**In** the present disclosure, the "treating" means all actions by which symptoms of a plant disease have taken a turn for the better or been modified favorably. **In** the present disclosure, the treating may include all actions by which infection with a pathogen or recurrence of a plant disease is prevented, symptoms are alleviated, the rate of progression is reduced, the disease state is relieved, the disease state is temporarily or continuously alleviated or mitigated, or the prognosis is improved.

**In** the present disclosure, the "plant disease" refers to a disease that occurs in a plant, and refers to abnormalities in its original appearance and physiological function. **The** cause of the disease may be disease injury caused by pathogens, nutrient deficiency in the soil, weather conditions, toxic gases, etc. **The** pathogen may be a fungal pathogen or a bacterial pathogen, but is not limited thereto.

**In** one embodiment, the plant disease of the present disclosure may be any one or more selected from the group consisting of Phytophthora stem and root rot, bacterial leaf blight, bacterial soft rot, rice blast, gray mold disease, leaf rust, powdery mildew, anthracnose, leaf spot (bacterial speck), damping off, sclerotinia rot, fusarium wilt, and wild fire, but is not limited thereto.

**In** the present disclosure, the "Phytophthora stem and root rot" is a type of fungal disease that occurs in potatoes or tomatoes, etc., and external symptoms are slightly different depending on the degree of infection. During the active growth period, the plant is stunted, the lower leaves appear reddish-brown, and young leaves may turn yellow to show various colors. When the infection is not severe, external symptoms are often unclear, and a diseased head produces small fruits and poor stolon. **The** Phytophthora stem and root rot may occur in potatoes, legumes, and fruit vegetables, but may occur without being limited to the type of plant. **The** Phytophthora stem and root rot is caused by a pathogen that is a fungus of the genus *Phytophthora* belonging to the family *Pythiaceae* of flagellated fungi, and may be specifically caused by *Phytophthora sojae, Phytophthora capsici,* and/or *Phytophthora infestans,* but is not limited thereto.

**In** one embodiment, the Phytophthora stem and root rot may be soybean Phytophthora stem and root rot, pepper Phytophthora stem and root rot, or potato Phytophthora stem and root rot, but is not limited thereto.

**In** the present disclosure, the "bacterial leaf blight" refers to a disease in which the disease pattern appears as spots and turns brown. **The** disease pattern may be formed on leaves, stems, pods, petioles, and cotyledons, etc. **It** may occur without being limited to the type of plant, such as beans, mung beans, or fruit vegetables, etc. **The** leaf blight is caused by a microorganisms of the genus *Pseudomonas,* specifically, *Pseudomonas syringae pv. lachrimans,* but is not limited thereto.

**In** one embodiment, the bacterial leaf blight may be cucumber bacterial leaf blight, but is not limited thereto.

**In** the present disclosure, the "bacterial soft rot" refers to a disease in which the plant tissue decay occurs to cause a foul odor and rotting. Particularly, it is common in juicy vegetables such as lettuce and napa cabbage, and also appears in tomatoes, sweet potatoes, and potatoes. **Thus,** bacterial soft rot may occur without being limited to the type of plant. **The** bacterial soft rot may be caused by a necrotrophic pathogen, specifically, *Pectobacterium carotovorum subsp. carotovorum,* but is not limited thereto.

**In** one embodiment, the bacterial soft rot may be lettuce bacterial soft rot, but is not limited thereto.

**In** the present disclosure, the "rice blast" is the most significant disease of grains, particularly, rice, and may occur in all parts of the grain. Lesions develop on above ground plant parts such as leaves, ears, ear branches, nodes, and rice grains, etc., but rice blast is the most common on leaves, ears, and ear branches. **It** is infected with conidia, and the common symptoms include red, gray, black, and/or brown irregular spots spreading on the leaves and the entire leaf turning brown and drying out. **The** ears or ear branches dry out to a light brown color and die, and when there is too much moisture, gray fungus grows on the surface. Dark brown disease patterns are formed at the nodes, and they are easily broken, and when the humidity is high, the surface may be covered with gray fungus. **The** rice blast may be caused by a microorganism of the genus *Magnaporthe,* specifically, *Magnaporthe oryzae,* but is not limited thereto.

**In** one embodiment, the rice blast may be rice blast in rice, but is not limited thereto.

**In** the present disclosure, the "gray mold disease" refers to a disease in which fungi invade plants and rot leaves, flowers, fruits, or branches. After remaining in the form of hyphae or sclerotia in debris such as leaves, flowers, fruits, or branches, etc., or in the soil, the petals and fruits decay to dark brown, and gray-green hyphae and spores may be formed. As the disease progresses, fruit drop and branch dieback may also appear. **The** gray mold disease may occur in citrus and tomatoes, but it may occur without being limited to the type of plant. **The** gray mold disease may be caused by a microorganism of the genus *Botrytis,* specifically, *Botrytis cinerea,* but is not limited thereto.

In one embodiment, the gray mold disease may be a tomato gray mold disease, but is not limited thereto.

In the present disclosure, the "leaf rust" is a disease that forms pustules on plants. It may occur not only on leaves but also on other parts such as stems, etc., and reddish-brown powdery mass may be formed on the pustules. There may be a slight difference in the lesion pattern and color, but as the lesions grow, they become fused and yellow, and if they progress, the plant will dry out and die. The leaf rust often occurs when crops grow well and prosper in the spring due to warm winters and high rainfall. The occurrence of the disease may also increase with early sowing and a lot of nitrogenous fertilizers. The leaf rust may occur in wheat, but is not limited thereto. The leaf rust may be caused by a microorganism of the genus *Puccinia,* specifically, *Puccinia hordei* or *Puccinia recondite,* but is not limited thereto.

In one embodiment, the leaf rust may be wheat leaf rust, but is not limited thereto.

In the present disclosure, the "powdery mildew" is a disease in which white or grayish-white fungi cover the surface of a plant body. The lesions may spread from the lower leaves to the leaf sheaths, stems, and even spikes, and in later stages, small black patches may appear on the grayish-white lesions. The occurrence may increase in warm, rainy years during the spring growing season, and it may also occur when plants are grown in places with poor ventilation or when grown with high nitrogenous fertilizer. The powdery mildew may occur in cereals such as barley, wheat, etc., but is not limited thereto. The powdery mildew may be caused by a microorganism of the genus *Blumeria,* specifically, *Blumeria graminis f. sp. Hordei,* but is not limited thereto.

In one embodiment, the powdery mildew may be barley powdery mildew, but is not limited thereto.

**In** the present disclosure, the "anthracnose" is a disease that mainly causes fruit rot. **It** may increase in hot and humid conditions such as the rainy season. Anthracnose may also occur on leaves and stems, but mainly occurs on fruits, causing light green spots on the fruits and even ulcer symptoms. **The** anthracnose may occur in a variety of plants, including fruit vegetables, citrus fruits, and legumes, etc., but is not limited thereto. **The** anthracnose may be caused by a microorganism of the genus *Colletotrichum,* specifically, *Colletotrichum coccodes, Colletotrichum truncatum,* and/or *Colletotrichum acutatum,* but is not limited thereto.

**In** one embodiment, the anthracnose may be pepper anthracnose, but is not limited thereto.

**In** the present disclosure, the "leaf spot" refers to a disease that causes large or small spots (or specks) on the stem, fruit, or leaves of a plant. **The** spots may be black, light brown, or brown. **The** leaf spot may occur in fruit vegetables, but it may occur without being limited to the type of plant. **The** leaf spot may be caused by several types of fungi or bacteria, and may be caused by a biotrophic pathogen, specifically, a microorganism of the genus *Pseudomonas,* more specifically, *Pseudomonas syringae pv. tomato,* much more specifically, *Pseudomonas syringae pv. tomato DC3000,* but is not limited thereto.

**In** the present disclosure, the "damping off" is a disease that causes a symptom of damping off. **It** may cause the symptom of damping off, rotting of seeds and non-germination, wilting of young seedlings after germination, or drying out symptoms, and/or rotting of root or stem, etc. **The** damping off may occur in fruit vegetables or root vegetables such as pepper, tomato, eggplant, watermelon, ginseng, legumes, cucumber, onion, and pepper, but is not limited to the type of plant. **The** damping off may be caused by a microorganism of the genus *Rhizoctonia* or *Pythium,* specifically, *Rhizoctonia solani* or *Pythium ultimum,* but is not limited thereto.

**In** the present disclosure, the "sclerotinia rot" is a disease in which pathogens remain in the state of sclerotia or hyphae in the plant and/or soil and gradually rot the plant. Hyphae grow at the site of infection, and soft rotting occurs and sclerotia may be formed. As it progresses, the lesions may expand and the entire plant may rot. Sclerotinia rot may occur in fruit vegetables or root vegetables such as cucumber, garlic, carrots, etc., but is not limited to the type of plant. **The** sclerotinia rot may be caused by a microorganism of the genus *Sclerotina,* specifically, *Sclerotina sclerotiorum,* but is not limited thereto.

**In** the present disclosure, the "fusarium wilt" is a disease that causes wilt symptoms. When the fusarium wilt occurs, plant growth may be inhibited, leaves may turn yellow, or root vascular parts may turn brown. **The** fusarium wilt may occur in fruit vegetables such as strawberries, but is not limited to the type of plant. **The** fusarium wilt may be caused by a microorganism of the genus *Fusarium,* specifically, *Fusarium oxysporum,* but is not limited thereto.

**In** the present disclosure, the "wild fire" is a disease that causes a plant to wither like wild fire in the field. As the disease progresses, spots may form and become necrotic, turning brown or black, or clusters of yellow lesions may appear on the plant. **The** wild fire may occur in tobacco or legumes, but is not limited to the type of plant. **The** wild fire may be caused by a microorganism of the genus *Pseudomonas,* specifically, *Pseudomonas syringae pv. tabaci,* but is not limited thereto.

**In** one embodiment, the plant disease may be caused by any one or more selected from the group consisting of *Phytophthora sojae, Pseudomonas syringae pv. lachrimans, Pectobacterium carotovorum subsp. Carotovorum, Magnaporthe oryzae, Botrytis cinerea, Puccinia recondite, Blumeria graminis f. sp. hordei, Colletotrichum coccodes, Pseudomonas syringae pv. tomato, Rhizoctonia solani, Pythium ultimum, Colletotrichum truncatum, Colletotrichum acutatum, Sclerotina sclerotiorum, Phytophthora capsici, Phytophthora infestans, Fusarium oxysporum,* and *Pseudomonas syringae pv. tabaci,* but is not limited thereto.

In one embodiment, the plant disease may be any one or more selected from the group consisting of Phytophthora stem and root rot caused by *Phytophthora sojae, Phytophthora capsici,* and/or *Phytophthora infestans,* bacterial leaf blight caused by *Pseudomonas syringae pv. lachrimans,* bacterial soft rot caused by *Pectobacterium carotovorum subsp. carotovorum,* rice blast caused by *Magnaporthe oryzae,* gray mold disease caused by *Botrytis cinerea,* leaf rust caused by *Puccinia recondite,* powdery mildew caused by *Blumeria graminis f. sp. Hordei,* anthracnose caused by *Colletotrichum coccodes, Colletotrichum truncatum,* and/or *Colletotrichum acutatum,* leaf spot caused by *Pseudomonas syringae pv. tomato,* damping off caused by *Rhizoctonia solani* and/or *Pythium ultimum,* sclerotinia rot caused by *Sclerotina sclerotiorum,* fusarium wilt caused by *Fusarium oxysporum,* and wild fire caused by *Pseudomonas syringae pv. tabaci,* but is not limited thereto.

The plant disease may occur in any type of plant, but in one embodiment, the plant may be selected from the group consisting of legumes, fruit vegetables, leafy vegetables, root vegetables, and grains.

In the present disclosure, the "legumes" refers to a plant of the family *Fabaceae* which is used as foods or feeds. For example, soybeans, wild soybeans, kidney beans, black beans, peas, red beans, peanuts, cowpeas, etc. may be included. Specifically, the legumes may be soybeans, but are not limited thereto.

In the present disclosure, the "fruit vegetables" refers to vegetables intended for the use of fruit, based on the part of the vegetable used. For example, peppers, cucumbers, tomatoes, watermelons, strawberries, oriental melons, etc. may be included. Specifically, the fruit vegetables may be peppers, cucumbers, or tomatoes, but are not limited thereto.

In the present disclosure, the "leafy vegetables" refers to vegetables intended for the use of leaves, based on the part of the vegetable used. For example, napa cabbage, lettuce, perilla leaves, spinach, etc. may be included. Specifically, the leafy vegetables may be napa cabbage or lettuce, but are not limited thereto.

**In** the present disclosure, the "root vegetables" refers to vegetables intended for the use of roots, based on the part of the vegetable used. For example, garlic and ginseng may be included, but are not limited thereto.

**In** the present disclosure, the "grains" refers to cultivated plants of the family *Gramineae* whose seeds are edible. For example, rice, wheat, barley, corn, etc. may be included. Specifically, the grains may be rice, wheat, or barley, but are not limited thereto.

**In** one embodiment, the composition for enhancing plant disease resistance may further include valine.

**The** valine may induce the plant immune system or may increase induced resistance. **In the** present disclosure, the composition for enhancing plant disease resistance including the N351 microorganism may further include valine, thereby further enhancing plant disease resistance while promoting induced resistance and growth, but is not limited thereto.

**In** one embodiment, the valine of the present disclosure may be included in an amount of 0.0001%(w/w) to 10%(w/w), 0.0001%(w/w) to 5%(w/w), 0.0001%(w/w) to 1%(w/w), 0.005%(w/w) to 10%(w/w), 0.005%(w/w) to 1%(w/w), 0.005%(w/w) to 0.5%(w/w), 0.001%(w/w) to 10%(w/w), 0.001%(w/w) to 1%(w/w), 0.001%(w/w) to 0.5%(w/w), 0.01 %(w/w) to 10%(w/w), 0.01 %(w/w) to 1%(w/w), 0.01%(w/w) to 0.5%(w/w), 0.05%(w/w) to 10%(w/w), 0.05%(w/w) to 1%(w/w), 0.05%(w/w) to 0.5%(w/w), 0.1%(w/w) to 10%(w/w), 0.1%(w/w) to 1%((w/w), or 0.1 %(w/w) to 0.5%(w/w) with respect to the total amount of the composition for enhancing plant disease resistance or the composition for controlling a plant disease, but is not limited thereto.

**The** composition for enhancing plant disease resistance of the present disclosure may include any agent or product needed to enhance plant disease resistance, such as pesticide preparations, fertilizers, control agents, etc., without limitation.

As used herein, the term "pesticide" refers to an agent used for the protection and growth of agricultural crops. Specifically, the pesticide may include preparations for use in controlling diseases, harmful insects, weeds, pathogens, nematodes, mites, etc. that occur during cultivation and storage of agricultural crops, as well as growth regulators for use in promoting or inhibiting the physiological functions of agricultural crops, and supplements for promoting remedial effects.

As used herein, the term "fertilizer", also called manure, refers to a nutrient that fertilizes the soil and promotes the growth of plants. A fertilizer including the composition of the present disclosure may be easily used by mixing with the soil before sowing, and may be applied together when spraying pesticides, etc.

**The** composition for enhancing plant disease resistance of the present disclosure may further include substances which are included for enhancing disease resistance commonly used. **In** addition to the N351 microorganism and valine, the composition may include, as an excipient, a pharmaceutically acceptable solid carrier, liquid carrier, liquid diluent, liquefied gas diluent, solid diluent or other auxiliaries, e.g., emulsifiers, dispersants, or surfactants such as foaming agents.

**The** composition for enhancing plant disease resistance of the present disclosure may be used after being formulated into an agricultural composition by mixing the active ingredient with the excipient, and as a method of formulating the agricultural composition, any method commonly used in the art may be applied.

Further, the composition for enhancing plant disease resistance of the present disclosure may be provided in the form of wettable powder, granules, powder, emulsion, spray, smoke agent, capsule, and gel, and may be provided in the form of a donut-shaped contact formulation for buoyancy of the composition, but is not limited to thereto.

Another aspect of the present disclosure provides a composition for controlling a plant disease, the composition including any one or more of the *Bacillus amyloliquefaciens* microorganism which is deposited with Accession No. KCCM13138P, and a culture thereof.

The *Bacillus amyloliquefaciens* microorganism which is deposited with Accession No. KCCM13138P, culture, and plant disease are as described in other aspects.

**In** one embodiment, the composition for controlling a plant disease may further include valine.

As used herein, the term "disease control" means preventing plants, such as crops, from being damaged by diseases and pests, removing pathogens that have occurred, or preventing the spread of pathogens. **The** disease control is a series of actions to reduce damage caused by pests, and may include physical (mechanical) control methods, agronomical control methods, chemical control methods, biological control methods, and eco-friendly control methods, but is not limited thereto. **The** disease control includes not only chemical control of using microbicides or insecticides which are toxic to pests, but also a method of creating an environment unsuitable for pathogens by cultivation of resistant plants, crop rotation, etc., a method of removing pathogens by heat treatment of plants or steam treatment of soil, a method of attracting and killing pests using attractants or sex pheromones.

**The** composition for controlling a plant disease of the present disclosure includes any agent or product needed to control the plant disease, such as pesticide preparations, fertilizers, control agents, etc., without limitation.

The composition for controlling a plant disease of the present disclosure may further include substances which are included for controlling a disease commonly used. In addition to the *Bacillus amyloliquefaciens* microorganism which is deposited with Accession No. KCCM13138P and valine, the composition may further include, as an excipient, a pharmaceutically acceptable solid carrier, liquid carrier, liquid diluent, liquefied gas diluent, solid diluent or other auxiliaries, e.g., emulsifiers, dispersants, or surfactants such as foaming agents.

The composition for controlling a plant disease of the present disclosure may be used after being formulated into an agricultural composition by mixing the active ingredient with the excipient, and as a method of formulating the agricultural composition, any method commonly used in the art may be applied.

Further, the composition for controlling a plant disease of the present disclosure may be provided in the form of wettable powder, granules, powder, emulsion, spray, smoke agent, capsule, and gel, and may be provided in the form of a donut-shaped contact formulation for buoyancy of the composition.

Still another aspect of the present disclosure provides a kit including the composition according to the present disclosure (i.e., the composition for enhancing plant disease resistance and/or for controlling a plant disease) and valine.

The composition according to the present disclosure, etc. are as described in other aspects.

The kit may be for controlling a plant disease or for preventing or treating a plant disease, but is not limited thereto. The kit may be provided such that the composition and valine may be stored in separate containers and mixed at the time of use, but is not limited thereto.

Further, the kit of the present disclosure may include one or more different components, compositions, solutions, or devices for preventing or treating a plant disease, for enhancing plant disease resistance, or for controlling a plant disease, but is not limited thereto. Examples of the different components may include, but are not limited to, appropriate carriers, solubilizers, buffers, stabilizers, etc. **The** carrier may include a soluble carrier or an insoluble carrier, and may be, for example, a physiologically acceptable buffer known in the art (e.g., PBS), but is not limited thereto.

Further, the kit of the present disclosure may further include a user guide that describes the conditions for performing the optimal reaction. **The** guide is a printout that explains how to use the kit, for example, reaction conditions, etc. **The** guide includes brochures in the form of pamphlets or leaflets, labels attached to the kit, and instructions on the surface of the package containing the kit. Further, the guide includes information disclosed or provided through electronic media such as the Internet.

Still another aspect of the present disclosure provides a method of enhancing plant disease resistance, the method including the step of treating a plant body with the composition for enhancing plant disease resistance including any one or more of a *Bacillus amyloliquefaciens* microorganism which is deposited with Accession No. KCCM13138P, and a culture thereof.

The *Bacillus amyloliquefaciens* microorganism which is deposited with Accession No. KCCM13138P, culture, plant disease, plant disease resistance, and composition for enhancing plant disease resistance are as described in other aspects.

**In** one embodiment, the composition for enhancing plant disease resistance may further include valine, but is not limited thereto.

**In** one embodiment, the method of enhancing plant disease resistance may promote plant growth, but is not limited thereto.

**The** plant body includes all of plant parts, such as roots, stems, leaves, or combinations thereof, and plant types, without limitation. Specifically, the plant body may be a plant individual at risk of being infected with a plant disease or infected with a pathogen, but is not limited thereto.

**The** treatment with the composition for enhancing plant disease resistance may be treatment by spraying, injecting, infusing, or mixing the composition to the plant body, or mixing the composition with the soil. However, as long as the inside or outside of the plant may be contacted with the composition directly or indirectly, it is not limited thereto. Further, the above treatment may be performed at the same time with or at the different time from invasion of a pathogen into the plant, and may be performed before or after invasion of a pathogen into the plant, but is not limited thereto.

**In** one embodiment, the composition for enhancing plant disease resistance may be treated once or twice or more, but is not limited thereto. When treated twice or more, it may be treated at intervals of 3 days to 15 days, 3 days to 10 days, 3 days to 7 days, 4 days to 7 days, 5 days to 7 days, or 6 days, but is not limited thereto.

**In** one embodiment, the composition for enhancing plant disease resistance may be treated to a plant body at a concentration of 1x10⁵ CFU/ml or more, 1x10⁶ CFU/ml or more, 1x10⁵ CFU/ml to 1x10¹⁰ CFU/ml, 1x10⁵ CFU/ml to 1x10⁹ CFU/ml, 1x10⁶ CFU/ml to 1x10¹⁰ CFU/ml, 1x10⁶ CFU/ml to 1x10⁹ CFU/ml, or 1x10³ CFU/ml to 1x10¹² CFU/ml, but is not limited thereto.

**In** one embodiment, the composition for enhancing plant disease resistance may be treated to a plant body in an amount of 1 ml to 50 ml, 2 ml to 50 ml, 3 ml to 50 ml, 4 ml to 50 ml, 5 ml to 50 ml, 1 ml to 30 ml, 2 ml to 30 ml, 3 ml to 30 ml, 4 ml to 30 ml, 5 ml to 30 ml, 1 ml to 20 ml, 2 ml to 20 ml, 3 ml to 20 ml, 4 ml to 20 ml, 5 ml to 20 ml, 1 ml to 10 ml, 2 ml to 10 ml, 3 ml to 10 ml, 4 ml to 10 ml, 5 ml to 10 ml, or 5 ml, but is not limited thereto.

**In** one embodiment, the plant may be any one or more selected from the group consisting of legumes, fruit vegetables, leafy vegetables, and grains, but is not limited thereto.

The legumes, fruit vegetables, leafy vegetables, and grains, etc. are as described in other aspects.

Still another aspect of the present disclosure provides a method of preventing or treating a plant disease, the method including the step of treating a plant body with the composition for enhancing plant disease resistance including any one or more of the *Bacillus amyloliquefaciens* microorganism which is deposited with Accession No. KCCM13138P and a culture thereof.

The *Bacillus amyloliquefaciens* microorganism which is deposited with Accession No. KCCM13138P, plant disease, plant disease resistance, composition for enhancing plant disease resistance, treatment with the composition for enhancing plant disease resistance, plant body, preventing, and treating, etc. are as described in other aspects.

Still another aspect of the present disclosure provides a method of controlling a plant disease, the method including the step of treating a plant body with the composition for controlling a plant disease including any one or more of the *Bacillus amyloliquefaciens* microorganism which is deposited with Accession No. KCCM13138P and a culture thereof. Specifically, the method of controlling a plant disease may promote plant growth.

The *Bacillus amyloliquefaciens* microorganism which is deposited with Accession No. KCCM13138P, culture, plant, plant body, plant disease, controlling, composition for controlling a plant disease, treatment with the composition for controlling a plant disease, etc. are as described in other aspects.

The treatment with the composition for controlling a plant disease may be treatment by spraying, injecting, infusing, or mixing the composition to the plant body, or mixing the composition with the soil. However, as long as the inside or outside of the plant may be contacted with the composition, it is not limited thereto. Further, the above treatment may be performed at the same time with or at the different time from invasion of a pathogen into the plant, and may be performed before or after invasion of a pathogen into the plant, but is not limited thereto.

**In** one embodiment, the composition for controlling a plant disease may be treated once or twice or more, but is not limited thereto. When treated twice or more, it may be treated at intervals of 3 days to 15 days, 3 days to 10 days, 3 days to 7 days, 4 days to 7 days, 5 days to 7 days, or 6 days, but is not limited thereto.

**In** one embodiment, the composition for controlling a plant disease may be treated to a plant body at a concentration of 1x10⁵ CFU/ml or more, 1x10⁶ CFU/ml or more, 1x10⁵ CFU/ml to 1x10¹⁰ CFU/ml, 1x10⁵ CFU/ml to 1x10⁹ CFU/ml, 1x10⁶ CFU/ml to 1x10¹⁰ CFU/ml, 1x10⁶ CFU/ml to 1x10⁹ CFU/ml, or 1x10³ CFU/ml to 1x10¹² CFU/ml, but is not limited thereto.

**In** one embodiment, the composition for controlling a plant disease may be treated to a plant body in an amount of 1 ml to 50 ml, 2 ml to 50 ml, 3 ml to 50 ml, 4 ml to 50 ml, 5 ml to 50 ml, 1 ml to 30 ml, 2 ml to 30 ml, 3 ml to 30 ml, 4 ml to 30 ml, 5 ml to 30 ml, 1 ml to 20 ml, 2 ml to 20 ml, 3 ml to 20 ml, 4 ml to 20 ml, 5 ml to 20 ml, 1 ml to 10 ml, 2 ml to 10 ml, 3 ml to 10 ml, 4 ml to 10 ml, 5 ml to 10 ml, or 5 ml, but is not limited thereto.

Still another aspect of the present disclosure provides a *Bacillus amyloliquefaciens* microorganism which is deposited with Accession No. KCCM13138P.

The *Bacillus amyloliquefaciens* microorganism which is deposited with Accession No. KCCM13138P is as described in other aspects.

Still another aspect of the present disclosure provides use of the *Bacillus amyloliquefaciens* microorganism which is deposited with Accession No. KCCM13138P in enhancing plant disease resistance, in controlling a plant disease, and in preventing or treating a plant disease.

Still another aspect of the present disclosure provides use of the composition including any one or more of the *Bacillus amyloliquefaciens* microorganism which is deposited with Accession No. KCCM13138P and a culture thereof, and valine in enhancing plant disease resistance, in controlling a plant disease, and in preventing or treating a plant disease.

The *Bacillus amyloliquefaciens* microorganism which is deposited with Accession No. KCCM13138P, culture, plant disease, plant disease resistance, controlling, preventing and treating, etc. are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification can be sufficiently understood and easily implemented by those skilled in the technical field of the present disclosure or similar technical fields.

### Example 1. Verification of control effect of Bacillus strain against plant disease

To verify the disease control effect of 16 types of the genus *Bacillus* strains, soybeans were used, and *Phytophthora sojae* (hereinafter, referred to as Ps) causing soybean Phytophthora stem and root rot was used as a pathogen.

To grow soybeans, the surface of seeds was sterilized by immersing the seeds in 3% sodium hypochlorite (NaOCl) for 5 minutes. Thereafter, the seeds were rinsed five times using sterile water and then sown in the bed soil (Fieldwan, Farm Hannong). 4 weeks after sowing, six leaves separated from the soybeans were placed on a square dish (125mmX125mm, SPL) covered with filter paper (110 mm, Watman). After placing a Ps pathogen disc (5 mm in diameter) on each leaf, each 500 µl of 16 types of the *Bacillus* strains (N474, N477, N481, N484, N485, N489, N490, N491, N494, N495, N311, N339, N343, N351, N352, and N429) were sprayed on the leaves at a concentration of 1X10⁸ cfu/ml.

16 types of the *Bacillus* strains were each cultured on a Luria-Bertani broth (LB) solid medium at 30°C for 24 hours. Ps pathogen was cultured in a V8-juice medium (200 ml; CaCO₃, 1 g; agar, 17 g/L) for 7 days. After measuring the length of symptoms on each of the six leaves 7 days after treatment with the *Bacillus* strain, the strain showing a statistical difference, as compared to a product control Serenade (Bayer)-treated group, was selected.

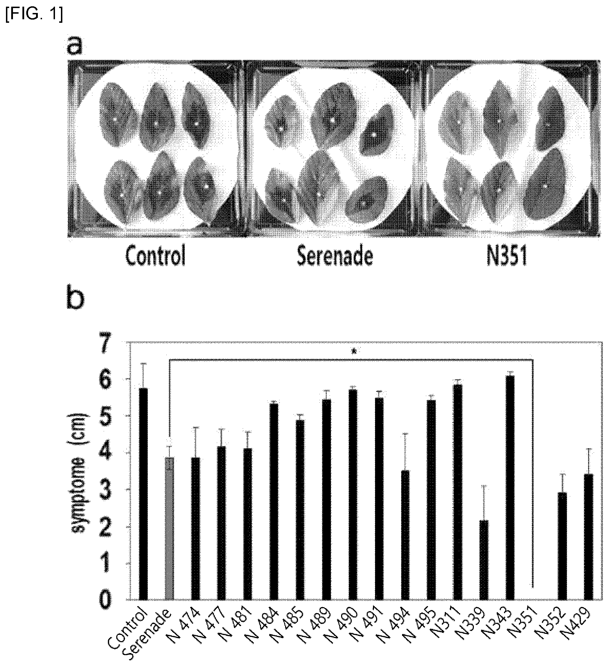

As shown in FIG. 1, as a result of comparing the control effects of 16 types of the strains-treated groups with those of the untreated control group and the product control group, the N351 strain-treated group showed the most excellent control effect, as compared to other groups treated with 15 types of the strains. FIG. 1a shows leaf images, and FIG. 1b shows the result of measuring the length of the symptom.

Most of the six leaves in the untreated group, the product control group, and the Bacillus-treated groups showed symptoms of turning brown due to Ps, but the N351 strain-treated group showed no symptoms 7 days even after Ps inoculation. The average length of symptoms in the product Serenade-treated group (product control group) was 3.9 cm. A total of 4 groups, N494, N339, N351, and N429-treated groups showed the shorter symptom length. Among them, only the N351 strain-treated group showed statistical significance, as compared to the Serenade-treated group (product control group).

The novel strain showing the most excellent control effect was named *Bacillus amyloliquefaciens* N351, and was deposited under the Budapest Treaty in the international depository authority, Korean Culture Center of Microorganisms on March 02, 2022, with Accession No. KCCM13138P.

### Example 2. Verification of microbicidal effect (plant disease control effect) of Bacillus amyloliquefaciens N351 strain against various plant pathogens

On Petri dishes with a diameter of 8.5 cm, each containing potato dextrose agar (PDA), V8 juice, Luria-Bertani broth (LB), and King's B (KB) medium, four paper discs were placed, and a reference strain *Bacillus siamensis (B. siamensis*^{T}*:* B. *siamensis* KACC15859; B.si), The paper discs were inoculated with a reference strain *Bacillus amyloliquefaciens (B. amyloliquefaciens*^{T}: *B. amyloliquefaciens* KACC 10116; B.a), *Bacillus subtilis (B. subtilis*^{T}: *B. subtilis* KACC 10854PB3; B.s), Serenade (Serenade identified from B. *velezensis* QST713), and three types of N351 strains. The remaining one paper disc was used as an untreated control group.

With regard to the fungal pathogens, the mycelium of the pathogen flora was placed on the center of the petri-dish using a cork borer with a diameter of 5 mm.

With regard to the bacterial pathogens (OD₆₀₀=1), 1000 µl thereof was spread on the petri-dish, and the strain of which microbicidal effect was intended to examine was inoculated on the paper disc. The strain to be examined was cultured on a Luria-Bertani broth (LB) solid medium at 30°C for 24 hours, and 50 µl of the strain (OD₆₀₀=1) was inoculated on the paper disc.

Among plant pathogens, *Rhizoctonia solani* (R.s), *Pythium ultimum* (P.u), *Colletotrichum truncatum* (C.t), *Colletotrichum acutatum* (C.a), *Sclerotina sclerotiorum* (S.s), *Phytophthora sojae* (P.s), *Phytophthora capsici* (P.c), *Phytophthora infestans* (P.i), *Fusarium oxysporum* (F.o), *Botrytis cinerea* (B.c), *Pectobacterium carotovorum subsp. Carotovorum* (Pcc), *Pseudomonas syringae pv. tabaci* (Pst), and *Pseudomonas syringae pv. lachrimans* (Psl), R.s, P.u, C.t, C.a, S.s, F.o, and B.c on a PDA medium, and P.s, P.c, and P.i on a V8 juice medium were cultured at 28°C for 5 days to 7 days. Pcc on an LB medium, and Pst and Psl strains on a KB medium were cultured at 28°Cfor 2 days.

Thereafter, each pathogen was cultured in an incubator at 25°C and 20°C for 7 days to 20 days, and then the diameter of the zone of inhibition was examined.

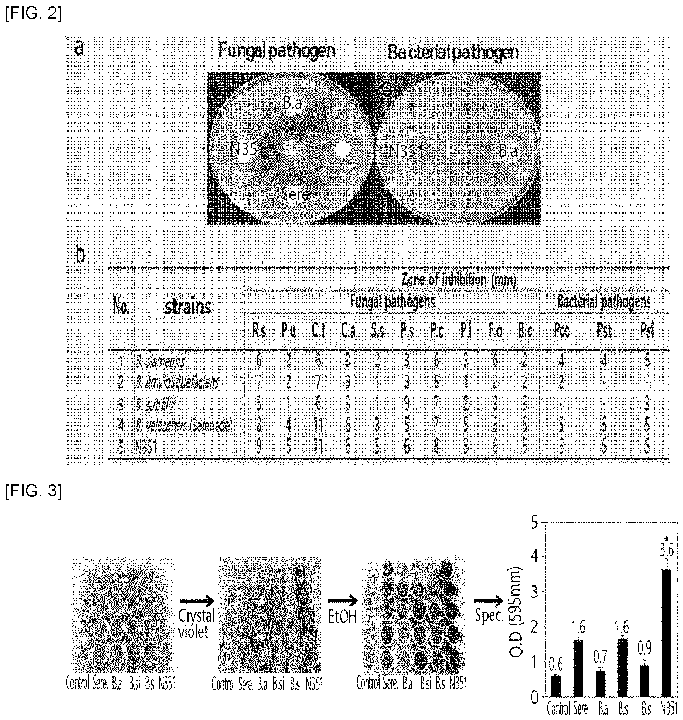

As a result, as shown in FIG. 2, the disease control effects of the N351 strain against various plant pathogens were verified.

### Example 3. Verification of biofilm of N351 strain

Microorganisms usually live in colonies to survive in their environment, and for this purpose, they form biofilms (film on the surface). As microorganisms well form biofilms, they well colonize all parts of the plant, and therefore, the biofilm is used as one of the criteria for selecting useful microorganisms.

In this Example, to measure the level of biofilm formation, a 96-well microplate made of polyvinyl chloride was used, and microorganisms with an absorbance of 0.05 at a wavelength of 600 nm were each inoculated in 100 ul of the medium per well, and cultured at 28°C for 2 days. To quantify the amount of biofilm, after culturing the microorganisms, 100 µl of 1% crystal violet solution was exposed to the microplate from which the supernatant was removed, for 15 minutes, and then excess crystal violet was removed with water. The crystal violet not removed by biofilm was eluted by adding 100 µl of 95% ethanol, and then the absorbance was measured at a wavelength of 595 nm using a microplate reader.

As a result, as shown in FIG. 3, the N351 strain produced the largest amount of biofilm, as compared to the four types of *Bacillus* strains which were compared and evaluated.

The N351 strain exhibited an absorbance value 6 times stronger than that of the untreated control. B.a and B.s exhibited absorbance similar to the untreated control group, and Serenade and B.si exhibited absorbance of 1.6, and formed only a small amount of biofilm. The N351 strain forms 2.3 times, 5 times, 2.3 times, and 4 times more biofilm than serenade, B.a, B.si, and B.s, respectively, confirming once again that the N351 strain shows the clear difference from the representative strains taxonomically belonging to the *Bacillus amyloliquefaciens* group *(Bacillus amyloliquefaciens, Bacillus siamensis,* and *Bacillus velezensis),* and also has a high potential as a biological control agent.

### Example 4. Verification of control effects of N351 strain and representative Bacillus strains

To compare and verify the control effects of the N351 strain and representative strains taxonomically belonging to the same group, a Ps pathogen disc was placed on a soybean leaf as in Example 1, and then each strain was sprayed. Thereafter, the degree of symptoms was measured using a hyperspectral camera and a visible light band camera.

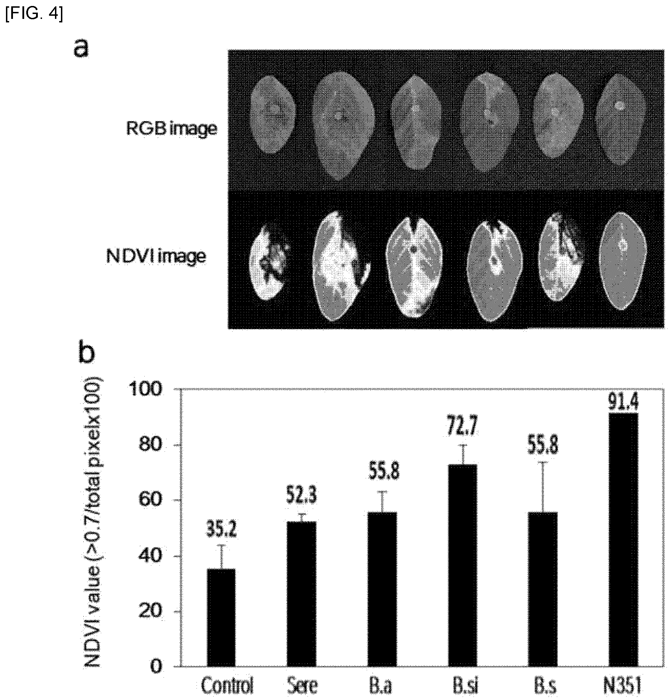

7 days after pathogen treatment, hyperspectral photographs were taken in the wavelength range of 400 nm to 1000 nm for each Bacillus treatment group (FIG. 4A). At this time, healthy and diseased leaves have different wavelength bands, which can be displayed in color. Even when it is difficult to distinguish differences in disease severity with the naked eye, using the hyperspectral camera makes it easy differentiate them. It was quantified by the normalized difference vegetation index (NDVI). The degree of diseased leaves in the entire leaf area was indicated from 0.1 to 0.9 and the number of the corresponding pixels was used. Generally, when the number is 0.7 or more, it is considered as a healthy leaf.

As a result, it was observed that in the untreated group , the green color faded and the disease progressed more quickly compared to all of the strain-treated group, even in the visible light band (FIG. 4A, RGB image). When photographed with the hyperspectral camera, leaves were photographed in different colors depending on the disease severity, ranging from healthy leaves (green) to dead leaves(brown) (FIG. 4A, NDVI image).

Since the N351 strain-treated group had healthy leaves of 91%, the proportion of healthy leaves was observed to be 56% higher than the untreated control group having healthy leaves of 35%. In contrast, other strain-treated groups, serenade, B.a, B.si, and B.s had merely 52%, 56%, 73%, and 56%, respectively, and accordingly, the excellent disease control effect of N351 against soybean Phytophthora stem and root rot was also observed through the hyperspectral camera (FIG. 4B).

### Example 5. Verification of control effect of N351 strain through pot experiment

The plant disease control effect of the N351 strain was verified on three crops grown in a square pot (5x5 cm).

Each crop was grown in a pot in a plant incubator (12-hour light culture, 28°C vision) for 3 weeks, and then sprayed with 5 ml of each *Bacillus* strain (OD₆₀₀=1) per plant. The *Bacillus* strain was used after being cultured at 30°C for 24 hours on an LB solid medium. 7 days after the first spray treatment, the second spray treatment was performed in the same manner as the first treatment, and one day later, pathogens were inoculated for each crop.

Phytophthora stem and root rot *(Phytophthora sojae,* P.s) for soybeans, bacterial leaf blight *(Pseudomonas syringae pv. lachrymans,* Psl) for cucumbers, and bacterial soft rot *(Pectobacterium carotovorum pv. carotovora,* Pcc) for lettuces were conducted.

P.s strain which is a pathogen of soybean Phytophthora stem and root rot was inoculated in a V8 medium (V8 juice, 1.8% agar), and cultured in an incubator at 25°C for 2 weeks, and the formed spores were harvested with sterile distilled water to prepare a spore suspension (5.0X10⁵ spores/ml) at a predetermined concentration. Soybeans, which were inoculated with the prepared spore suspension, were allowed to develop the disease for 3 days in a constant temperature and humidity room at a temperature of 26°C, and 7 days after treatment with the pathogen, the disease severity was scored from 0 to 5, based on the following criteria.
- 0=no symptoms, 1=1 foliage leaf withered, 2=2 foliage leaves withered, 3=3 foliage leaves withered, 4=4 foliage leaves withered, and 5=severe withering of the entire plant.

Psl strain which is a pathogen of cucumber bacterial leaf blight was cultured on a King's B (KB) solid medium at 28°C for 48 hours. **The** pathogen was treated at a concentration of OD=1 (10⁹ CFU/ml). **The** cucumbers inoculated with the pathogen were allowed to develop the disease for 3 days in a constant temperature and humidity room at a temperature of 26°C, and 7 days after treatment with the pathogen, the disease severity was scored from 0 to 5, based on the following criteria.
- 0=no symptoms, 1=mild disease symptoms, 2=30% disease symptoms, 3=disease symptoms and mild necrosis, 4=necrosis and 5=severe necrosis and withering.

Pcc strain which is a pathogen of lettuce bacterial soft rot was cultured on a Luria-Bertani broth (LB) solid medium at 28°C for 48 hours. **The** pathogen was treated at a concentration of OD=1 (10⁹ CFU/ml). **The** lettuces inoculated with the pathogen were allowed to develop the disease for 1 day in a constant temperature and humidity room at a temperature of 26°C, and 48 hours after treatment with the pathogen, the disease severity was observed and scored from 0 to 5, based on the following criteria.
- 0=no symptoms, 1=slight soft rot symptoms at the pathogen infection site, 2=soft rot symptoms at the pathogen infection site, 3=severe soft rot symptoms at the pathogen infection site, 4=soft rot symptoms around the pathogen infection site, 5=soft rot symptoms throughout the leaf.

The control effect (control value) against soybean Phytophthora stem and root rot, cucumber bacterial leaf blight, and lettuce bacterial soft rot in the N351 strain-treated group, as compared to the untreated group, was calculated using the disease severity as follows.

| | |
|---|---|
| - | Control value (%) = (1 - Disease severity of treated group/Disease severity of untreated group)X100 |

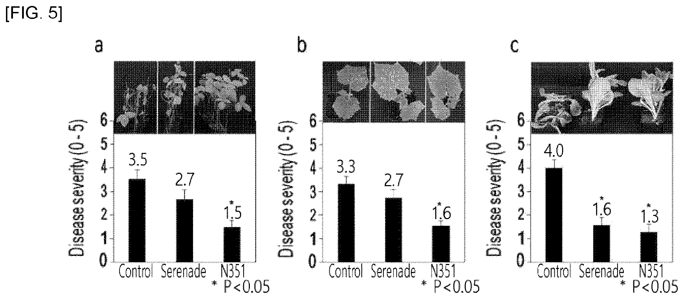

With regard to soybean Phytophthora stem and root rot, the disease severity in the N351 strain-treated group was 1.5, which was lowered, as compared to 3.5 in the water control group. The control value was calculated as 57%, compared to the untreated group. The N351 strain-treated group showed the excellent disease control effect, as compared to the serenade-treated group, which had a control value of 23% (FIG. 5A).

With regard to cucumber bacterial leaf blight, the disease severity in the N351 strain-treated group was 1.6, which was lowered, as compared to 3.3 in the water control group. The control value was calculated as 52%, compared to the untreated group. The N351 strain-treated group showed the excellent disease control effect, as compared to the serenade-treated group, which had a control value of 18% (FIG. 5B).

With regard to lettuce bacterial soft rot, the disease severity in the N351 strain-treated group was 1.3, which was greatly lowered, as compared to 4 in the water control group. The control value was calculated as 68%, compared to the untreated group. The N351 strain-treated group showed the excellent disease control effect, similar to the serenade-treated group, which had a control value of 60% (FIG. 5C).

### Example 6. Verification of control effect of N351 strain powder preparation through pot experiment

The N351 strain was prepared in a powder form in order to observe whether the N351 strain also exhibits the excellent control effects against plant diseases even when it was prepared into a powder preparation.

To this end, the N351 strain was cultured on an LB solid medium at 28°C for 24 hours, and a single colony was inoculated into an LB liquid medium. The liquid medium was cultured at 28°C and 200 rpm for 48 hours. The culture medium was centrifuged (4,000 rpm, 20 minutes) to be separated into bacterial cells and a culture filtrate. Diatomaceous earth was added in an amount equal to the weight of the bacterial cells, warm-dried at 30°C for 3 days, and powdered.

The prepared N351 powder preparation (1X10⁹ CFU/g) was diluted 500-times, and sprayed onto seedlings of each plant grown and prepared in a greenhouse, and 1 day later, each of the five plant pathogens was inoculated.

Five pathogens of rice blast *(Magnaporthe oryzae),* tomato gray mold *(Botrytis cinerea),* wheat leaf rust *(Puccinia recondite),* barley powdery mildew *(Blumeria graminis f. sp. hordei),* and pepper anthracnose *(Colletotrichum coccodes)* were inoculated into a total of five crops.

*Magnaporthe oryzae* KI-1113a strain which is a pathogen of rice blast (RCB) was inoculated on a rice polish agar, and cultured in an incubator at 25°C for 2 weeks, and the formed spores were harvested with sterile distilled water to prepare a spore suspension (5.0X10⁵ spores/ml) at a predetermined concentration. Rices, which were inoculated with the spore suspension, were left in the dark in a humid room for 24 hours, and allowed to develop the disease for 4 days in a constant temperature and humidity room with a relative humidity of 80% at a temperature of 25°C, and then a diseased area (%) was examined.

*Botrytis cinerea* strain which is a pathogen of tomato gray mold (TGM) was inoculated on a potato agar medium, and cultured in an incubator (dark condition) at 20°C, and the formed spores were used as an inoculum. For pathogen inoculation, spores were harvested and the spore concentration was prepared at 5.0X10⁵ spores/ml using a hemocytometer, followed by spraying onto the sample-treated tomato seedlings (2^{nd} to 3^{rd} leaf stage). The inoculated tomato seedlings were left in a humid room (relative humidity of 95% or more) at 20°C to develop the disease for 3 days, and then a diseased area (%) was examined.

*Puccinia recondita* which is a pathogen of wheat leaf rust (WLR) is a biotrophic pathogen, and thus while subculturing directly on the plants in the laboratory, spores (urediniospores) formed on wheat leaves were used as an inoculum. To investigate the medicinal efficacy, each of 5 grains of wheat seeds ('Geumgang wheat') was sown in disposable pots (diameter: 4.5 cm), and 1-leaf seedlings grown in a greenhouse for 8 days were treated with samples, and 1 day later, the inoculum (0.67 g/L spores) was inoculated by spraying. The inoculated wheat seedlings were treated in a humid room at 20°C for 1 day, and then transferred to a constant temperature and humidity room with a relative humidity of 70% at 20°C to induce disease development. 7 days after inoculation, a diseased area (%) was examined.

*Blumeria graminis f. sp. Hordei* which is a pathogen of barley powdery mildew (BPM) is a biotrophic pathogen, and thus while subculturing barley seedlings in the laboratory, spores formed on barley leaves were used as an inoculum. To investigate the medicinal efficacy, each of 5 grains of barley seeds ('Hanyoung barley') was sown in disposable pots (diameter: 4.5 cm), and 1-leaf seedlings grown in a greenhouse for 8 days were treated with samples, and air-dried in a greenhouse, and powdery mildew spores were shaken off and inoculated into the sample-treated barley. The inoculated barley seedlings were left in a constant temperature and humidity room with a relative humidity of 50% at 20°C to induce disease development for 7 days. Then, a diseased area (%) was examined.

*Colletotrichum coccodes* which is a pathogen of pepper anthracnose (PAN) was inoculated on an oatmeal medium, and cultured at 25°C for 10 days to form spores. The formed spores were harvested and adjusted to a spore concentration of 5X10⁵ spores/ml to prepare a spore suspension. The prepared spore suspension was spray-inoculated onto sample-treated pepper seedlings (3^{rd} to 4^{th} leaf stage), and left in a humid room (25°C) and treated in a humid for 2 days, and then were allowed to develop the disease in a constant temperature and humidity room (25°C 80% RH). 3 days after inoculation, a diseased area (%) on the pepper leaves was examined. The diseased areas obtained from disease examination and control values using the same were calculated according to the following Equation.

| | |
|---|---|
| - | Diseased area = Sum of area of diseased leaves/Sum of total leaf area examined X 100% |
| - | Control value (%) = (1 - Diseased area of treated group/Diseased area of untreated group)X100 |

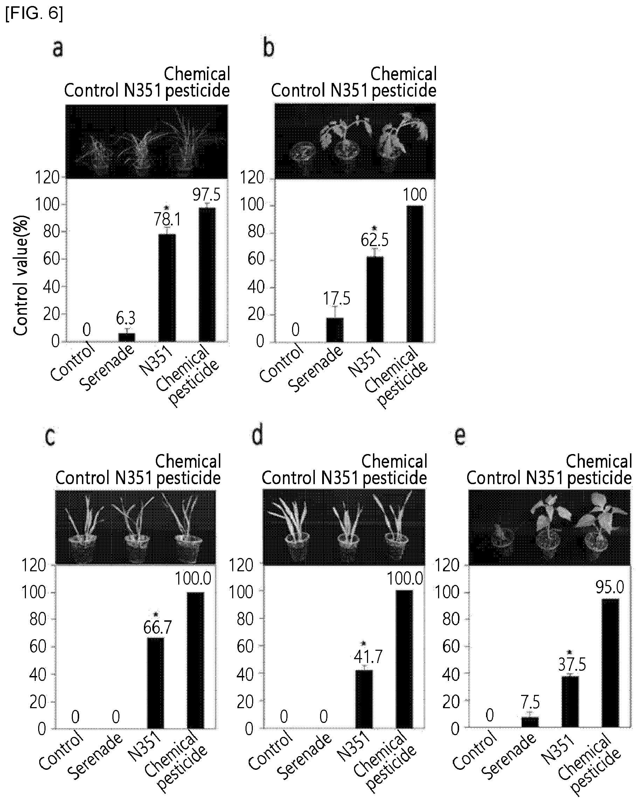

As a result, as shown in FIG. 6, it was found that when the control effect of the N351 powder preparation was tested for 5 pathogens, it had the excellent control effect on all the five diseases, as compared to the product control group.

With respect to rice blast (FIG. 6A), the N351 powder preparation-treated group showed a high control value (78%), as compared to the untreated control group (0%), and a control value 13 times higher than the product control group (6%).

With respect to tomato gray mold (FIG. 6B), the N351 powder preparation-treated group showed a high control value (63%), as compared to the untreated control group (0%), and a control value 3.6 times higher than the product control group (18%).

With respect to wheat leaf rust (FIG. 6C), the N351 powder preparation-treated group showed a high control value (67%), as compared to the untreated control group (0%). In contrast, the product control group showed no control effect.

With respect to barley powdery mildew (FIG. 6D), the N351 powder preparation-treated group showed a high control value (42%), as compared to the untreated control group (0%). In contrast, the product control group showed no control effect, like on wheat leaf rust.

With respect to pepper anthracnose (FIG. 6E), the N351 powder preparation-treated group showed a high control value (38%), as compared to the untreated control group (0%), and a control value 5 times higher than the product control group (8%).

The above results confirmed that the N351 strain powder preparation showed a control value of 3.6 times or more for five diseases, as compared to the product control group.

### Example 7. Verification of control effect of mixed treatment of N351 powder preparation and valine through pot experiment

To further enhance the plant disease control effect of the N351 powder preparation, which has the better control effect than the product control group, valine was used together. By mixed treatment of the N351 powder preparation and valine, it was attempted to maximize the comprehensive control effect by sterilizing pathogens and increasing plant immunity.

To this end, the disease control effect of the mixed treatment of the N351 powder preparation and valine was investigated with respect to the two crops grown in a pot (square pot. 5x5 cm). When treated together with the N351 powder preparation, valine was used at a concentration of 0.05%. The treatment was performed on Phytophthora stem and root rot (Phytophthora sojae) of soybeans, and bacterial leaf blight *(Pseudomonas syringae pv. lachrymans)* of cucumbers. Plant preparation of soybeans and cucumbers, preparation of *Bacillus* strain, preparation of pathogens of soybean Phytophthora stem and root rot and cucumber bacterial leaf blight, and induction of disease development were performed in the same manner as in Example 5. The pathogens were treated at a concentration of OD=1 (10⁹ CFU/ml). The inoculated soybeans and cucumbers were allowed to develop the disease in a constant temperature and humidity room at a temperature of 26°C for 3 day, and then disease severity was observed. The control value was calculated as follows.

| | |
|---|---|
| - | Control value (%) = (1 - Disease severity of treated group/Disease severity of untreated group) X 100 |

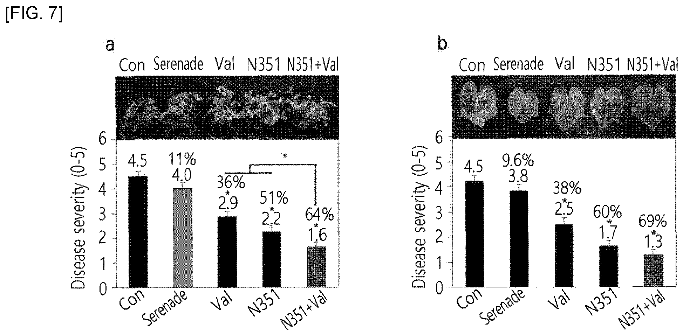

As a result, as shown in FIG. 7, it was observed that when the control effect of the mixed treatment of the N351 powder preparation and valine was investigated for soybean Phytophthora stem and root rot and cucumber bacterial leaf blight, its control effect was improved, as compared to the single treatment group.

With respect to soybean Phytophthora stem and root rot, serenade, valine, and N351 powder preparation-treated groups showed a control value (%) of 11%, 36%, and 51%, respectively, as compared to the untreated group, whereas the mixed treatment group of the N351 powder preparation and valine showed a control value of 64%, which is 13% higher than that of the N351 powder preparation-treated group. The control value of the mixed treatment group of the N351 powder preparation and valine was statistically significantly higher than each single treatment group. The control value of the N351 powder preparation-treated group was 40% higher than that of the product control group, whereas the control value of the mixed treatment group of the N351 powder preparation and valine was 53% higher (FIG. 7A).

With respect to cucumber bacterial leaf blight, serenade, valine, and N351 powder preparation-treated groups showed a control value (%) of 10%, 38%, and 60%, respectively, as compared to the untreated group, whereas the mixed treatment group of the N351 powder preparation and valine showed a control value of 69%, which is 10% higher than that of the N351 powder preparation-treated group. Even though the control value of the N351 powder preparation-treated group was 50% higher than that of the product control group, the mixed treatment group of the N351 powder preparation and valine improved the control value by 59% (FIG. 7B).

### Example 8. Verification of control effect of mixed treatment of N351 powder preparation and valine through glass greenhouse

In the pot experiment which was conducted on soybeans and cucumbers in a plant incubator, it was observed that the plant disease control effect of mixed treatment of the N351 powder preparation and valine was superior to the single treatment group, and thus it was examined whether the disease control effect was also reproduced even in a glass greenhouse environment. The subject of the experiment was soybeans. Plant preparation, treatment with N351 powder preparation and valine, and treatment of soybean Phytophthora stem and root rot were performed in the same manner as Example 7. After treatment of Phytophthora stem and root rot, the disease was allowed to develop in a constant temperature and humidity room at 28°C in a glass greenhouse for 2 days, and disease severity was observed. The control value was calculated as follows.

| | |
|---|---|
| - | Control value (%) = (1 - Disease severity of treated group/Disease severity of untreated group) X 100 |

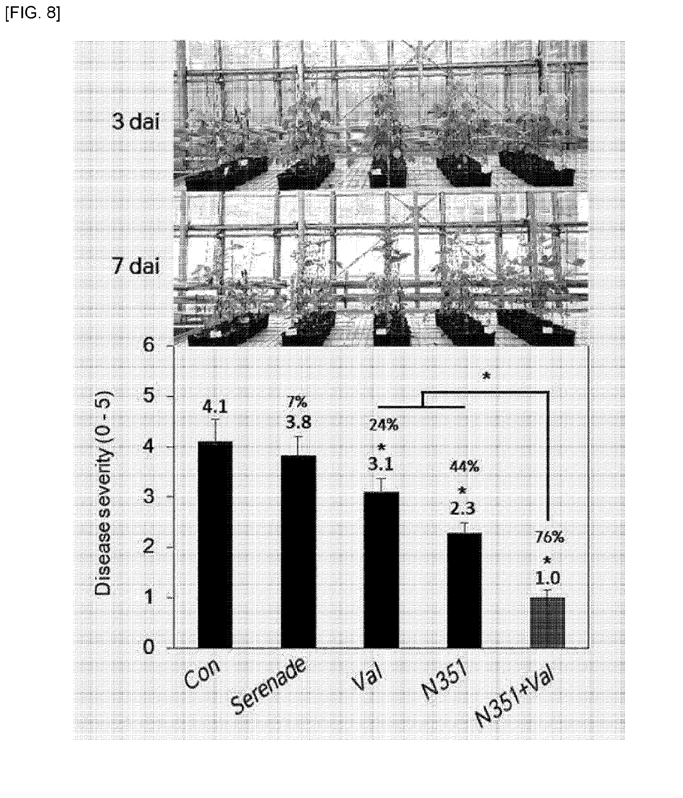

As a result, as shown in FIG. 8, when the control effect of mixed treatment of the N351 powder preparation and valine was investigated for soybean Phytophthora stem and root rot, the control effect improved as compared to the single treatment group was also observed in the glass greenhouse.

With respect to soybean Phytophthora stem and root rot, serenade, valine, and N351 powder preparation-treated groups showed a control value (%) of 7.3%, 24%, and 44%, respectively, as compared to the untreated group, whereas the mixed treatment group of the N351 powder preparation and valine showed a control value of 76%, indicating that the control value may be more improved through mixed treatment than single treatment. The control value of the mixed treatment group of the N351 powder preparation and valine was statistically significantly higher than each single treatment group.

### Example 9. Verification of induced resistance of mixed treatment of N351 powder preparation and valine

The effect of inducing induced resistance by mixed treatment of N351 powder preparation and valine was verified.

To this end, *Arabidopsis thaliana* which is a plant model was used, and *Pseudomonas syringae pv. tomato* DC3000 (hereinafter, referred to as Pst) causing leaf spot was used as a pathogen. To grow the plant, the surface of seeds of *Arabidopsis thaliana* was sterilized by immersing the seeds in 3% sodium hypochlorite (NaOCl) for 5 minutes. Thereafter, the seeds were rinsed five times using sterile water and then sown in a pot, and cultured in a plant incubator (12-hour light and 23°C temperature conditions) for 3 weeks, followed by root zone treatment with each 50 ml of benzothiadiazole (BTH), serenade, N351 strain, valine, and mixture of N351 strain and valine. 7 days after treatment, the pathogen was inoculated and the population of pathogens was examined 0 day and 3 days later. To examine the population of pathogens, the leaves were cut into 1 cm discs using a cork borer and placed in an e-tube containing 1 ml of sterile water and beads. The leaves were ground for 2 minutes at 6000 rpm using a homogenizer (Bertin), and the population of pathogens in the juice was examined.

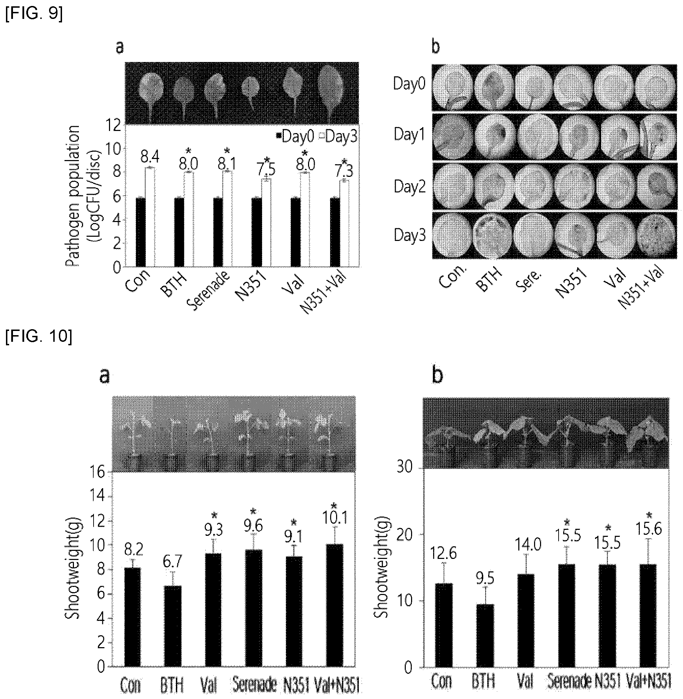

As a result, as shown in FIG. 9A, as a result of measuring the population of pathogens on 3 days after treating the *Arabidopsis thaliana* plant body with the Pst pathogen, the population of pathogens in the mixed treatment group of N351 powder preparation and valine was statistically significant reduced, as compared to the untreated control group. The mixed treatment group of N351 powder preparation and valine showed 7.3 LogCFU/disc, indicating a better pathogen inhibition effect than the N351 powder preparation-treated group of 7.5 LogCFU/disc and the valine-treated group of 8 LogCFU/disc, and it was observed that the N351 strain, valine, and their combination have antibacterial activity as well as induced resistance.

Additionally, to determine the mechanism by which the mixed treatment of N351 powder preparation and valine induces induced resistance, the expression of a disease resistance-related gene was investigated. *Arabidopsis thaliana* containing Pathogenesis-related protein (PR) 1a promoter GUS fusion construct which is a disease resistance marker gene was used. Preparation of *Arabidopsis thaliana,* treatment with N351 powder preparation and valine, and pathogen treatment were performed in the same manner as above. To observe the expression of PR1a, plant leaf samples were prepared 0 day, 1 day, 2 days, and 3 days after disease treatment.

As a result, as shown in FIG. 9B, it was observed that GUS activity by the mixed treatment of N351 powder preparation and valine was expressed in the leaf area from 1 day after disease treatment to 3 days after observation. In the single treatment group of N351 powder preparation, GUS expression was observed from 1 day to 3 days after disease treatment, and the expression was stronger in the mixed treatment group. In the single treatment group of valine, GUS expression was most strongly induced 1 day after disease treatment, and expression became weaker over time. When the mixed treatment of N351 powder preparation and valine was performed, it was observed that GUS expression strongly occurred from the beginning due to valine, and persisted by the N351 powder preparation.

### Example 10. Verification of growth promoting ability of mixed treatment of N351 powder preparation and valine

The growth of soybeans and cucumbers was observed by mixed treatment of N351 powder preparation and valine.

One week after sowing in pots, root zone treatment of each plant was performed using each 5 ml of BTH, valine, N351 powder preparation, and the mixture of N351 powder preparation and valine. The root zone treatment with BTH, valine, N351 powder preparation, and the mixture of N351 powder preparation and valine was performed twice at one week intervals, respectively. One week after the last treatment, a shoot of each treatment group was cut and weighed.

As a result, in the case of soybeans, as shown in FIG. 10A, the groups treated with the N351 powder preparation, valine, and the mixture of N351 powder preparation and valine were weighed 9.1 g, 9.3 g, and 10.1 g on average, respectively, indicating that they further promoted growth by 0.9 g, 1.1 g, and 1.9 g, as compared to the untreated group of 8.2 g. In contrast, growth of the BTH-treated group which is an induced resistance product control was rather inhibited, as compared to the untreated control group. It was confirmed that growth promotion was statistically significantly induced in all the groups, excluding the BTH-treated group, as compared to the untreated control group.

In the case of cucumbers, as shown in FIG. 10B, the groups treated with the N351 powder preparation, valine, and the mixture of N351 powder preparation and valine were weighed 15.5 g, 14 g, and 15.6 g on average, respectively, indicating that they further promoted growth by 2.9 g, 1.4 g, and 3 g, as compared to the untreated group of 12.6 g. Similar to the soybeans, the mixed treatment group showed the most excellent promotion effect, but the strain seemed to have a greater effect than valine. The BTH-treated group also showed growth inhibition in cucumbers, as compared to the untreated control group. The mixed treatment group of N351 powder preparation and valine induced statistically significant growth promotion.

**BTH** (benzo(1,2,3)-thiadiazile-7-carbothioic acid S-methyl ester, benzothiadiazole) is known as the most effective derivative inducing pathogen resistance in plants. However, serious problems were found, such as inhibition of plant growth or significant reduction of yield by **BTH** treatment.

The above results confirmed that treatment with N351 or the mixture of N351 and valine did not inhibit plant growth, but rather promoted plant growth.

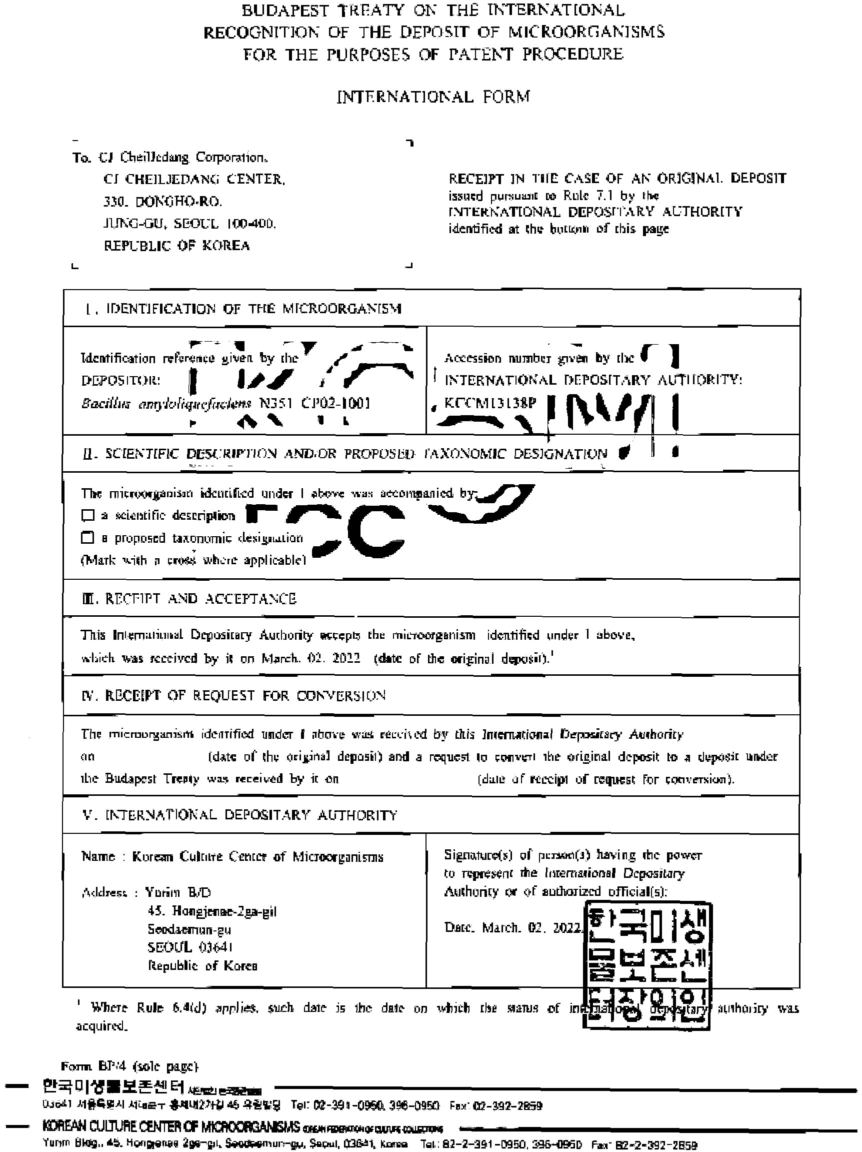
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation.
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I . IDENTIFICATION OF THE MICROORGANISM
Identification reference given by the
DEPOSITOR:
Bacillus amyloliquefaciens N351 CP02-1001 | Accession number given by the
INTERNATIONAL DEPOSITARY AUTHORITY:
KCCM13138P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on March. 02. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date. March. 02. 2022.

¹ Where Rule 6.4(d) applies, such date is the date on which the status of international depositary authority was
acquired.

Form BP/4 (sole page)
한국미생물보존센터 [?]
03641 서울특별시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea Tel: 82-2-391-0950, 396-0950 Fax: 82-2-392-2859

## Claims

1. Use of a composition for enhancing plant disease resistance, the composition comprising any one or more of a *Bacillus amyloliquefaciens* microorganism which is deposited with Accession No. KCCM13138P and a culture thereof.

2. **The** use according to claim 1, wherein the plant disease is any one or more selected from the group consisting of Phytophthora stem and root rot, bacterial leaf blight, bacterial soft rot, rice blast, gray mold disease, leaf rust, powdery mildew, anthracnose, leaf spot (bacterial speck), damping off, sclerotinia rot, fusarium wilt, and wild fire.

3. **The** use according to claim 1, wherein the composition is for any one or more selected from the group consisting of legumes, fruit vegetables, leafy vegetables, root vegetables, and grains.

4. **The** use according to claim 1, wherein the plant disease is caused by any one or more pathogens selected from the group consisting of *Phytophthora sojae, Pseudomonas syringae pv. lachrimans, Pectobacterium carotovorum subsp. Carotovorum, Magnaporthe oryzae, Botrytis cinerea, Puccinia recondite, Blumeria graminis f. sp. hordei, Colletotrichum coccodes, Pseudomonas syringae pv. tomato, Rhizoctonia solani, Pythium ultimum, Colletotrichum truncatum, Colletotrichum acutatum, Sclerotina sclerotiorum, Phytophthora capsici, Phytophthora infestans, Fusarium oxysporum,* and *Pseudomonas syringae pv. tabaci.*

5. **The** use according to claim 1, wherein the composition prevents or treats a plant disease;
wherein the composition induces systemic induced resistance of plants; or wherein the composition induces plant growth promotion.

6. The use according to claim 1, wherein the composition further comprises valine.

7. Use of a composition for controlling a plant disease, the composition comprising any one or more of a *Bacillus amyloliquefaciens* microorganism which is deposited with Accession No. KCCM13138P and a culture thereof, optionally
wherein the composition further comprises valine.

8. A method of enhancing plant disease resistance, the method comprising the step of treating a plant body with a composition for enhancing plant disease resistance comprising any one or more of a *Bacillus amyloliquefaciens* microorganism which is deposited with Accession No. KCCM13138P and a culture thereof.

9. The method of claim 8, wherein the plant is any one or more selected from the group consisting of legumes, fruit vegetables, leafy vegetables, root vegetables, and grains.

10. The method of claim 8, wherein the composition further comprises valine.

11. A method of preventing or treating a plant disease, the method comprising the step of treating a plant body with a composition for enhancing plant disease resistance comprising any one or more of a *Bacillus amyloliquefaciens* microorganism which is deposited with Accession No. KCCM13138P and a culture thereof.

12. The method of claim 11, wherein the composition further comprises valine.

13. A method of controlling a plant disease, the method comprising the step of treating a plant body with a composition for controlling a plant disease comprising any one or more of a *Bacillus amyloliquefaciens* microorganism which is deposited with Accession No. KCCM13138P and a culture thereof.

14. The method of claim 13, wherein the composition further comprises valine.

15. *A Bacillus amyloliquefaciens* microorganism which is deposited with Accession No. KCCM13138P, optionally wherein the microorganism has any one or more of a plant disease resistance-enhancing ability and a plant disease-controlling ability.

## Patentansprüche

1. Verwendung einer Zusammensetzung zum Verstärken einer Pflanzenkrankheitsresistenz, wobei die Zusammensetzung einen oder mehrere der unter der Zugangsnummer KCCM13138P hinterlegten Bacillus amyloliquefaciens-Mikroorganismen und eine Kultur davon umfasst.

2. Verwendung nach Anspruch 1, wobei es sich bei der Pflanzenkrankheit um eine oder mehrere handelt, die aus der Gruppe bestehend aus Phytophthora-Stamm- und Wurzelfäule, bakterieller Blattfleckenkrankheit, bakterieller Nassfäule, Reisbrand, Grauschimmelkrankheit, Blattrost, Echter Mehltau, Anthraknose, Blattfleckenkrankheit (Tüpfelschwärze), Umfallkrankheit, Sklerotinia-Fäule, Fusarium-Welke und Feuerbrand ausgewählt sind.

3. Verwendung nach Anspruch 1, wobei die Zusammensetzung für eines oder mehrere aus der Gruppe bestehend aus Hülsenfrüchten, Fruchtgemüse, Blattgemüse, Wurzelgemüse und Getreide vorgesehen ist.

4. Verwendung nach Anspruch 1, wobei die Pflanzenkrankheit durch einen oder mehrere Krankheitserreger verursacht wird, die aus der Gruppe bestehend aus Phytophthora sojae, Pseudomonas syringae pv. lachrimans, Pectobacterium carotovorum subsp. Carotovorum, Magnaporthe oryzae, Botrytis cinerea, Puccinia recondite, Blumeria graminis f. sp. hordei, Colletotrichum coccodes, Pseudomonas syringae pv. tomato, Rhizoctonia solani, Pythium ultimum, Colletotrichum truncatum, Colletotrichum acutatum, Sclerotina sclerotiorum, Phytophthora capsici, Phytophthora infestans, Fusarium oxysporum und Pseudomonas syringae pv. tabaci ausgewählt sind.

5. Verwendung nach Anspruch 1, wobei die Zusammensetzung eine Pflanzenkrankheit verhindert oder behandelt,
wobei die Zusammensetzung eine systemische induzierte Resistenz von Pflanzen hervorruft, oder
wobei die Zusammensetzung eine Förderung des Pflanzenwachstums induziert.

6. Verwendung nach Anspruch 1, wobei die Zusammensetzung ferner Valin umfasst.

7. Verwendung einer Zusammensetzung zum Kontrollieren einer Pflanzenkrankheit, wobei die Zusammensetzung einen oder mehrere der unter der Zugangsnummer KCCM13138P hinterlegten Bacillus amyloliquefaciens-Mikroorganismen und eine Kultur davon umfasst, wobei die Zusammensetzung optional ferner Valin umfasst.

8. Verfahren zum Verstärken einer Pflanzenkrankheitsresistenz, wobei das Verfahren den Schritt des Behandelns eines Pflanzenkörpers mit einer Zusammensetzung zum Verstärken einer Pflanzenkrankheitsresistenz umfasst, wobei die Zusammensetzung einen oder mehrere der unter der Zugangsnummer KCCM13138P hinterlegten Bacillus amyloliquefaciens-Mikroorganismen und eine Kultur davon umfasst.

9. Verfahren nach Anspruch 8, wobei es sich bei der Pflanze um eine oder mehrere aus der Gruppe bestehend aus Hülsenfrüchten, Fruchtgemüse, Blattgemüse, Wurzelgemüse und Getreide handelt.

10. Verfahren nach Anspruch 8, wobei die Zusammensetzung ferner Valin umfasst.

11. Verfahren zum Verhindern oder Behandeln einer Pflanzenkrankheit, wobei das Verfahren den Schritt des Behandelns eines Pflanzenkörpers mit einer Zusammensetzung zum Verstärken einer Pflanzenkrankheitsresistenz umfasst, wobei die Zusammensetzung einen oder mehrere der unter der Zugangsnummer KCCM13138P hinterlegten Bacillus amyloliquefaciens-Mikroorganismen und eine Kultur davon umfasst.

12. Verfahren nach Anspruch 11, wobei die Zusammensetzung ferner Valin umfasst.

13. Verfahren zum Kontrollieren einer Pflanzenkrankheit, wobei das Verfahren den Schritt des Behandelns eines Pflanzenkörpers mit einer Zusammensetzung zum Verstärken einer Pflanzenkrankheitsresistenz umfasst, wobei die Zusammensetzung einen oder mehrere der unter der Zugangsnummer KCCM13138P hinterlegten Bacillus amyloliquefaciens-Mikroorganismen und eine Kultur davon umfasst.

14. Verfahren nach Anspruch 13, wobei die Zusammensetzung ferner Valin umfasst.

15. Bacillus amyloliquefaciens-Mikroorganismus, der unter der Zugangsnummer KCCM13138P hinterlegt ist, wobei der Mikroorganismus optional eine oder mehrere einer Pflanzenkrankheitsresistenz-verstärkenden Fähigkeit und einer pflanzenkrankheitskontrollierten Fähigkeit aufweist.

## Revendications

1. Utilisation d'une composition destinée à améliorer la résistance des plantes aux maladies, la composition comprenant un ou plusieurs des éléments suivants : un micro-organisme Bacillus amyloliquefaciens qui est déposé sous le numéro d'accès KCCM13138P, et une culture de celui-ci.

2. L'utilisation selon la revendication 1, dans laquelle la maladie des plantes est une ou plusieurs choisies parmi le groupe constitué de la pourriture des tiges et des racines causée par Phytophthora, de la brûlure bactérienne des feuilles, de la pourriture molle bactérienne, de la pyriculariose du riz, de la pourriture grise, de la rouille des feuilles, de l'oïdium, de l'anthracnose, de taches foliaires (taches bactériennes), de la fonte des semis, de la pourriture sclérotique, de la flétrissure fusarienne et du feu sauvage.

3. L'utilisation selon la revendication 1, dans laquelle la composition est destinée à un ou plusieurs éléments choisis parmi le groupe comprenant les légumineuses, les légumineuses fruitières, les légumineuses à feuilles, les légumineuses à racines et les céréales.

4. L'utilisation selon la revendication 1, dans laquelle la maladie végétale est causée par un ou plusieurs agents pathogènes choisis parmi le groupe comprenant Phytophthora sojae, Pseudomonas syringae pv. lachrimans, Pectobacterium carotovorum subsp. Carotovorum, Magnaporthe oryzae, Botrytis cinerea, Puccinia recondita, Blumeria graminis f. sp. hordei, Colletotrichum coccodes, Pseudomonas syringae *pv. tomato, Rhizoctonia solani, Pythium ultimum, Colletotrichum truncatum, Colletotrichum acutatum, Sclerotina sclerotiorum, Phytophthora capsici, Phytophthora infestans, Fusarium oxysporum, et Pseudomonas syringae pv. tabaci.*

5. L'utilisation selon la revendication 1, dans laquelle la composition prévient ou traite une maladie des plantes, dans laquelle la composition induit une résistance systémique induite chez les plantes; ou dans laquelle la composition induit une résistance systémique induite chez les plantes.

6. L'utilisation selon la revendication 1, dans laquelle la composition comprend en outre de la valine.

7. Utilisation d'une composition destinée à lutter contre une maladie des plantes, la composition comprenant un ou plusieurs des éléments suivants : un micro-organisme Bacillus amyloliquefaciens qui est déposé sous le numéro d'accès KCCM13138P et une culture de celui-ci, dans laquelle la composition comprend en outre, de manière facultative, de la valine.

8. Procédé visant à améliorer la résistance des plantes aux maladies, le procédé comprenant l'étape consistant à traiter un corps végétal avec la composition destinée à améliorer la résistance des plantes aux maladies comprenant un ou plusieurs des éléments suivants : un micro-organisme Bacillus amyloliquefaciens qui est déposé sous le numéro d'accès KCCM13138P, et une culture de celui-ci.

9. Procédé selon la revendication 8, dans lequel la plante est une ou plusieurs plantes choisies parmi le groupe comprenant les légumineuses, les légumineuses fruitières, les légumineuses à feuilles, les légumineuses à racines et les céréales.

10. Procédé selon la revendication 8, dans laquelle la composition comprend en outre de la valine.

11. Procédé de prévention ou de traitement d'une maladie des plantes, le procédé comprenant l'étape consistant à traiter un corps végétal avec la composition destinée à améliorer la résistance des plantes aux maladies comprenant un ou plusieurs des éléments suivants : un micro-organisme Bacillus amyloliquefaciens qui est déposé sous le numéro d'accès KCCM13138P, et une culture de celui-ci.

12. Procédé selon la revendication 11, dans laquelle la composition comprend en outre de la valine.

13. Procédé de lutte contre une maladie des plantes, le procédé comprenant l'étape consistant à traiter un corps végétal avec une composition destinée à lutter contre une maladie des plantes comprenant un ou plusieurs des éléments suivants : un micro-organisme Bacillus amyloliquefaciens qui est déposé sous le numéro d'accès KCCM13138P, et une culture de celui-ci.

14. Procédé selon la revendication 13, dans laquelle la composition comprend en outre de la valine.

15. Micro-organisme Bacillus amyloliquefaciens déposé sous le numéro d'accès KCCM13138P, dans lequel le micro-organisme présente, de manière facultative, une ou plusieurs des propriétés suivantes : une capacité à renforcer la résistance des plantes aux maladies et une capacité à lutter contre les maladies des plantes.
